# EUROPÄISCHE PATENTANMELDUNG

(11) **EP 0 661 238 A1**
(43) Veröffentlichungstag der Anmeldung: **05.07.1995**
(21) Anmeldenummer: 94108027.7
(22) Anmeldetag: 25.05.1994
(51) Int. Cl.: C02F 3/28, C02F 1/58, C05F 3/00

(54) **Verfahren und Vorrichtung zur Entsorgung von Biomasse**

(30) Priorität: 04.12.1993 DE 4341713
(71) Anmelder: Flüh, Gerd, D-21339 Lüneburg (DE); Huber, Peter, D-21438 Brackel (DE)
(72) Erfinder: Flüh, Gerd, D-21339 Lüneburg (DE); Huber, Peter, D-21438 Brackel (DE)
(74) Vertreter: Vonnemann, Gerhard, Dr.-Ing.

(57) **Zusammenfassung**

Es wird ein verfahren zur Entsorgung von Biomasse, insbesondere Gülle beschrieben, mit welchem mit besonders hoher Ausbeute Biogas erzeugt werden kann und somit eine wirtschaftliche Energiegewinnung möglich ist. Die Biomasse wird dazu anaerob vergoren, wobei während der Vergärung zumindest einem Teil des Biomasse Ammoniak entzogen wird.

## Beschreibung

Die Erfindung betrifft ein Verfahren zur Entsorgung von Biomasse, insbesondere Gülle, sowie eine Vorrichtung zur Durchführung des Verfahrens.

Die Entsorgung von Biomasse, insbesondere Gülle, ist in vielen landwirtschaftlichen Betrieben zu einem Problem geworden. Auf Äckern kann die Gülle nur in begrenzten Mengen verteilt werden, da ansonsten zahlreiche unerwünschte Folgen eintreten. Ein Teil der Gülle versickert entweder ungenutzt im Boden und belastet das Grundwasser, oder gelangt mit dem Regenwasser in Flüsse und Seen und führt dort zu einer Überdüngung mit den bekannten Problemen. Weiterhin wird aber auch die Luft durch die in der Gülle enthaltenen flüchtigen Stickstoff-Verbindungen belastet.

Aus der DE-PS 39 21 241 ist ein Verfahren zum Entsorgen von Gülle bekannt, bei dem durch Verdünnung von Gülle eine Nährlösung hergestellt wird, in der Pflanzen gehalten werden. Die Pflanzen nehmen die in der Gülle enthaltenen Stoffe, insbesondere Nitrate, Nitrite, Phosphate und Kalzium auf und geben im wesentlichen Wasser an die Nährlösung ab. Der mit diesem Verfahren verbundene Aufwand ist offensichtlich und überhaupt nur dann sinnvoll, wenn es sich bei den Pflanzen um Nutzpflanzen handelt.

Ferner ist es auch bekannt (DE-OS 29 04 335 und DE-OS 34 00 529), die in Abwassern enthaltenen Stickstoffverbindungen mit Hilfe von Mikroorganismen abzubauen, wobei der entstehende Abwasserschlamm mit thermischen, mechanischen oder chemischen Verfahren weiterbehandelt werden muß.

Der Erfindung liegt demgegenüber die Aufgabe zugrunde, ein Verfahren zur Entsorgung von Biomasse, insbesondere Gülle, anzugeben, mit dem eine sehr wirtschaftliche Ausnutzung der in der Biomasse enthaltenen Energie möglich ist.

Die Lösung dieser Aufgabe erfolgt bei dem eingangs genannten Verfahren dadurch, daß die Biomasse zur Gewinnung von Biogas anaerob vergoren wird und während der Vergärung zumindest einem Teil der Biomasse Ammoniak entzogen wird.

Diese Lösung hat den Vorteil, daß die Ausbeute des bei der anaeroben Vergärung entstehenden Biogases wesentlich verbessert ist. Durch die Entziehung des Ammoniaks während der Vergärung wird nämlich die hemmende Wirkung dieses Stoffes auf die das Biogas erzeugenden Mikroorganismen aufgehoben.

Das Biogas, welches im wesentlichen aus Methan besteht, verbrennt sehr sauber und belastet dabei die Umwelt kaum.

Das entzogene Ammoniak kann bei einer bevorzugten Ausführungsform des Verfahrens der vergorenen Biomasse oder einer Teilmenge dann wieder zugesetzt werden. Die auf diese Weise angereicherte Biomasse wird dann der Endlagerung für Düngezwecke zugeführt, so daß auch bezüglich der Entsorgung des Ammoniaks keine Probleme entstehen.

Die Biomasse wird zum Entzug des Ammoniaks vorzugsweise auf eine Temperatur oberhalb der Vergärungstemperatur erwärmt. Das Abscheiden des Ammoniaks kann mit Wasserdampf erfolgen, was insbesondere dann besonders wirkungsvoll ist, wenn diese Abscheidung unter Vakuum erfolgt. Das dabei entstehende Ammoniak-Wasserdampf-Gemisch kann entweder kondensiert oder von der vergorenen Biomasse absorbiert werden.

Die Wirksamkeit des Verfahrens kann weiter gesteigert werden, wenn die Abscheidung des Ammoniaks mehrstufig erfolgt.

Weiterhin kann in einem Kreisprozeß Biomasse ständig einem ersten Vergärungstank entnommen, erwärmt und nach der Abscheidung von Ammoniak und Abkühlung auf Vergärungstemperatur diesem Tank wieder zugeführt werden (nicht kontinuierliche Verfahrensweise).

Alternativ dazu kann bei einem kontinuierlichen Verfahren die gesamte Biomasse dem ersten Vergärungstank entnommen und erwärmt werden und nach Abscheidung von Ammoniak und Abkühlung auf Vergärungstemperatur einem zweiten Vergärungstank zugeführt werden.

Weitere Einzelheiten, Merkmale und Vorteile der Erfindung ergeben sich aus der nachfolgenden Beschreibung von Ausführungsbeispielen anhand der Zeichnungen.

Es zeigt:
- Fig. 1: den prinzipiellen Ablauf einer ersten Ausführungsform des erfindungsgemäßen Verfahrens und
- Fig. 2: den prinzipiellen Ablauf einer zweiten Ausführungsform des erfindungsgemäßen Verfahrens.

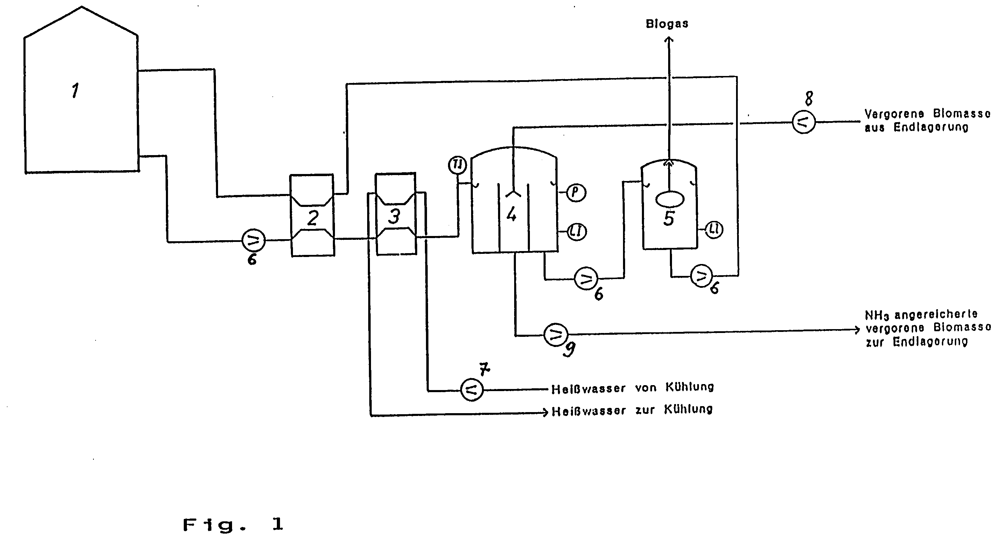

Gemäß Figur 1 ist ein erster Vergärungstank 1 zur Aufnahme und Vergärung unvergorener Biomasse vorgesehen. Die Vergärung erfolgt anaerob, das heißt unter Abschluß von Luft-Sauerstoff mit Hilfe von Mikroorganismen. Während dieses Vorganges wird ein Teilstrom Biomasse aus dem ersten Vergärungstank 1 entnommen und über eine erste Umwälzpumpe 3 und einen ersten Wärmetauscher 4 geführt. In diesem Wärmetauscher wird die Biomasse erwärmt, während gleichzeitig der in den ersten Vergärungstank zurückfließende Teilstrom wieder nahezu auf Vergärungstemperatur abgekühlt wird, so daß das Verfahren sehr wirtschaftlich arbeitet. Zur weiteren Erwärmung und zur Bereitstellung der Verdampfungswärme wird der Teilstrom nun über einen zweiten Wärmetauscher 5 geleitet, dem über eine zweite Umwälzpumpe 6 Heißwasser zugeführt wird. Dieses Wasser kann beispielsweise von dem entstehenden Methan oder dem Kühlwasser einer Kraft-Wärme-Kopplung beheizt worden sein.

Nachdem der Biomassen-Teilstrom die gewünschte Temperatur erreicht hat, gelangt er in einen Ammoniakabscheider 7. Das in der Biomasse enthaltene Ammoniak wird dort unter Vakuum als Ammoniak-Wasserdampf-Gemisch ausgetrieben. Mit diesem Gemisch wird vergorene kältere Biomasse angereichert, die dem Ammoniakabscheider 7 aus einer Endlagerung über eine Förderpumpe 9 zugeführt wird. Nachdem die abgekühlte und vergorene Biomasse das Ammoniak absorbiert hat, wird sie über eine Pumpe 10 wieder dem Endlager zugeführt. Die ammoniakärmere Biomasse verläßt, ebenso wie das Biogas, den Ammoniakabscheider 7 über eine vierte Umwälzpumpe 8 und wird anschließend dem ersten Wärmetauscher 4 zugeführt, dort vorgekühlt und dann im dritten Wärmetauscher 11 mittels unvergorener Biomasse, die durch Leitung 12 zu- bzw. durch Leitung 13 abfließt, auf Vergärungstemperatur abgekühlt und in den ersten Vergärungstank 1 zurückgeleitet. Leitung 12 zieht beipielsweise mittels einer Pumpe Gülle aus einem Stall ab, während Leitung 13 diese Gülle nach ihrer Erwärmung dem Vergärungstank 1 zuführt. Dieser Wärmetauscher hat den Vorteil, daß er einen größeren Temperaturunterschied zwischen Primär- und Sekundärseite aufweist, als Wärmetauscher 4. Dieser zuvor beschriebene Kreisprozeß wird solange aufrechterhalten, bis die Vergärung der Biomasse einen optimalen Wert erreicht hat.

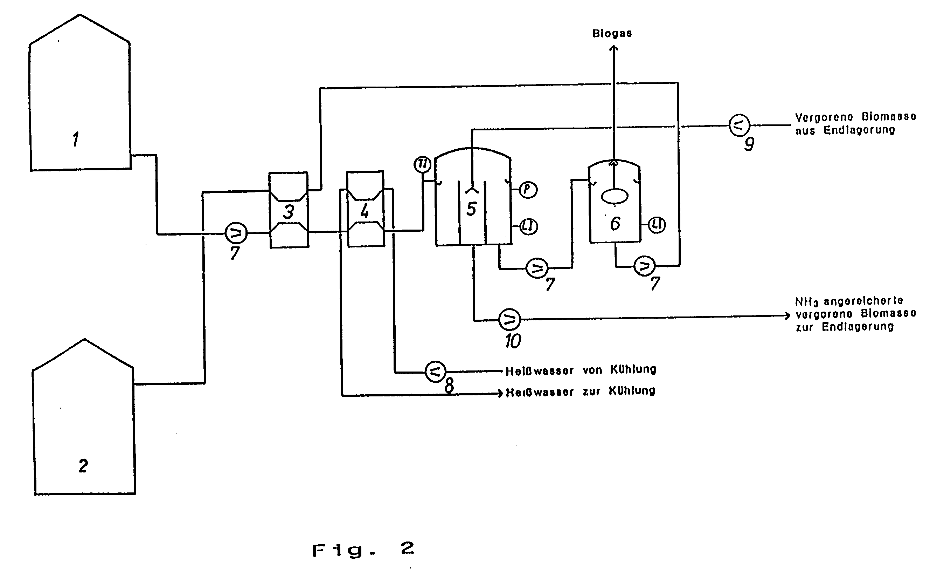

Eine Abscheidung der Mikroorganismenbiomasse ist nicht vorgesehen. Am Ende der Fermentation sterben diese ab und gehen in Autolyse über. Die verbleibenden organischen Verbindungen verbleiben in der ausgegorenen Gülle. Die Temperaturführung ist so gewählt, daß eine Pasteurisation der Gülle eintritt. Dies ist besonders wichtig bei dem Verfahren gemäß Figur 2, um eine Sperre für Bakteriophagen zwischen Vergärungstanks 1 und 2 zu haben.

Figur 2 zeigt eine zweite Ausführungsform des Verfahrens, bei dem Biomasse und Biogas nach Abscheidung des Ammoniaks nicht wieder in den ersten Vergärungstank 1 zurückgeführt, sondern in einen zweiten Vergärungstank 2 geleitet werden. Auf diese Weise wird kontinuierlich die gesamte Biomasse erwärmt, einer Ammoniakabscheidung unterzogen, abgekühlt und anschließend dem zweiten Vergärungstank zugeführt, der eine nächste Vergärungsstufe darstellt. Im übrigen ist der Ablauf der gleiche, wie der in Figur 1 gezeigte.

### BEZUGSZEICHENLISTE

- 1: erster Vergärungstank
- 2: zweiter Vergärungstank
- 3: erste Umwälzpumpe
- 4: erster Wärmetauscher
- 5: zweiter Wärmetauscher
- 6: zweite Umwälzpumpe
- 7: Ammoniakabscheider
- 8: dritte Umwälzpumpe
- 9: vierte Umwälzpumpe
- 10: fünfte Umwälzpumpe
- 11: dritter Wärmetauscher
- 12: unvergorene Biomasse von Rezeption
- 13: unvergorene Biomasse zur Vergärung

## Patentansprüche

1. Verfahren zur Entsorgung von Biomasse, **dadurch gekennzeichnet,** daß die Biomasse zur Gewinnung von Biogas anaerob vergoren wird und daß während der Vergärung zumindest einem Teil der Biomasse Ammoniak entzogen wird.

2. Verfahren nach Anspruch 1, **dadurch gekennzeichnet,** daß die Biomasse Gülle ist.

3. Verfahren nach Anspruch 1, **dadurch gekennzeichnet,** daß der Gülle andere organische Abfälle zugesetzt werden.

4. Verfahren nach mindestens einem der vorhergehenden Ansprüche, **dadurch gekennzeichnet,** daß das entzogene Ammoniak kondensiert wird.

5. Verfahren nach mindestens einem der vorhergehenden Ansprüche, **dadurch gekennzeichnet,** daß die Biomasse zum Entzug des Ammoniaks erwärmt wird.

6. Verfahren nach mindestens einem der vorhergehenden Ansprüche, **dadurch gekennzeichnet,** daß das Ammoniak mit Wasserdampf entzogen wird.

7. Verfahren nach Anspruch 5, **dadurch gekennzeichnet,** daß das Ammoniak unter Vakuum ausgetrieben wird.

8. Verfahren nach Anspruch 6, **dadurch gekennzeichnet,** daß das Ammoniak-Wasserdampf-Gemisch kondensiert und von vergorener Biomasse absorbiert wird.

9. Verfahren nach mindestens einem der vorhergehenden Ansprüche, **dadurch gekennzeichnet,** daß die Abscheidung von Ammoniak mehrstufig erfolgt.

10. Verfahren nach mindesten einem der vorhergehenden Ansprüche, **dadurch gekennzeichnet,** daß in einem Kreisprozeß Biomasse einem ersten Vergärungstank (1) entnommen, vorzugsweise erwärmt und nach der Abscheidung von Ammoniak sowie vorzugsweiser Abkühlung auf Vergärungstemperatur dem ersten Vergärungstank wieder zugeführt wird.

11. Verfahren nach mindestens einem der Ansprüche 1 bis 10, **dadurch gekennzeichnet,** daß die Biomasse nach Abscheidung von Ammoniak in einen zweiten Vergärungstank (2) geführt wird.

12. Vorrichtung zur Durchführung des Verfahrens nach mindestens einem der vorhergehenden Ansprüche, **gekennzeichnet durch** einen ersten Vergärungstank (1), einen ersten Wärmetauscher (4) zur Erwärmung eines Teilstromes von Biomasse und einen Ammoniakabscheider (7).

13. Vorrichtung nach Anspruch 12, **gekennzeichnet durch** einen zweiten Vergärungstank (2) zur Aufnahme von Biomasse nach Entzug des Ammoniaks.
